(19) Europäisches Patentamt
European Patent Office
Office européen des brevets

(11) Numéro de publication : **0 067 086**
**B1**

(12) # FASCICULE DE BREVET EUROPÉEN

(45) Date de publication du fascicule du brevet : **11.12.85**

(21) Numéro de dépôt : **82400862.7**

(22) Date de dépôt : **10.05.82**

(51) Int. Cl.⁴ : **C 07 D313/08**, C 07 D337/08, C 07 D223/16, A 61 K 31/335, A 61 K 31/38, A 61 K 31/55// C07C59/72, C07C69/734

(54) **Nouveaux composés de la série des benzoxépines et analogues soufrés et azotés, leurs procédés et utilisations en tant que médicament.**

(30) Priorité : **11.05.81 FR 8109327**
**04.05.82 FR 8207693**

(43) Date de publication de la demande :
**15.12.82 Bulletin 82/50**

(45) Mention de la délivrance du brevet :
**11.12.85 Bulletin 85/50**

(84) Etats contractants désignés :
**AT BE CH DE FR GB IT LI LU NL SE**

(56) Documents cités :
**US-A- 3 332 951**
**US-A- 3 444 176**
**COLLECTION OF CZECHOSLOVAK CHEMICAL COMMUNICATIONS, vol. 43, pub. en 1978, pages 1760-77, Prague (CS); M. RAJSNER et al.: "4,4-Bis(4-Fluorophenyl)butylamines and their cyclic analogues; an efficient synthesis of the neuroleptic penfluridol"**
**JOURNAL OF MEDICINAL CHEMISTRY, vol. 10, avril 1967, pages 575-82, Columbus Ohio (USA); L.H. WERNER et al.: "Imidazoline derivatives with antiarrhythmic activity"**
**CHEMICAL ABSTRACTS, vol. 83, no. 19, 10 novembre 1975, page 529, no. 163961s, Columbus Ohio (USA); V.N. GOGTE et al.: "Synthesis of heterocyclic compounds. XIV. Interaction of dimethylsulfoxonium methylide with quinolin N-oxide and 7-methoxy-4-phenylquinoline N-oxide"**

(73) Titulaire : **PIERRE FABRE S.A.**
**125, rue de la Faisanderie**
**F-75116 Paris (FR)**

(72) Inventeur : **Hatinguais, Philippe**
**Le Landou Chemin de Bel Air**
**F-81100 Castres (FR)**
Inventeur : **Patoiseau, Jean-François**
**112, rue Paul Gaugin**
**F-81100 Castres (FR)**
Inventeur : **Marcelon, Gilbert**
**Route de Castres Sémalens**
**F-81700 Puylaurens (FR)**

(74) Mandataire : **Martin, Jean-Jacques et al**
**Cabinet REGIMBEAU 26, Avenue Kléber**
**F-75116 Paris (FR)**

**EP 0 067 086 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

COLLECTION OF CZECHOSLOVAK CHEMICAL COMMUNICATIONS, vol. 37, pub. en 1972, pages 2081-90, Prague (CS); M. PROTIVA et al.: "Benzocycloheptenes and heterocyclic analogues as potential drugs. IV. Amines derived from 4-Phenyl-8-Chloro-2, 3,4,5-Tetrahydro-1-Benzoxepin"
CHEMICAL ABSTRACTS, vol. 92, no. 1, 7 janvier 1980, page 599, no. 6363s, Columbus Ohio (USA); B. PIERFRANCESCO et al.: "Oxygen heterocycles by sulfur ylide annulation. 2,3,4,5-Tetrahydro-3-hydroxy-1-benzooxepins from 2-(0-hydroxyphenyl) alkyl ketones and dimethyloxosulfonium methylide"

## Description

La présente invention concerne de nouveaux composés de la série des benzoxépines et analogues soufrés et azotés.

Cette invention concerne également les procédés pour la préparation de ces produits et l'application de ceux-ci dans le domaine pharmaceutique.

Bien que les benzoxépines constituent une classe chimique comprenant de nombreux composés, aucun des composés connus n'a été décrit comme présentant des propriétés pharmacologiques dans le domaine cardiovasculaire.

La Demanderesse a découvert que les phényl-3 dihydro-2,3 benzoxépines-1 et leurs analogues dans les séries benzazépine et benzothiépine, composés chimiques nouveaux, sont dotés de précieuses propriétés dans le domaine cardiovasculaire.

La présente invention concerne des composés de formule :

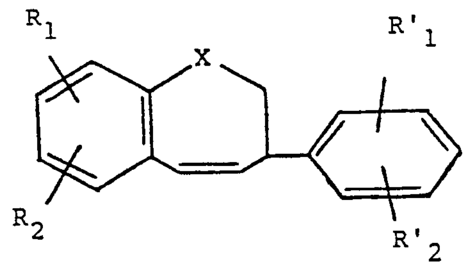

(I)

dans laquelle :

X est l'oxygène, le soufre ou $>$N-R', R' étant l'hydrogène ou un radical $C_1$-$C_5$ alkyle ;

$R_1$, $R_2$, $R'_1$ et $R'_2$ représentent indépendamment :

un atome d'hydrogène ou d'halogène,

un radical $C_1$-$C_5$-alkyle, $C_5$-$C_{12}$-aryle, $C_1$-$C_5$-alcoxy, $C_5$-$C_{12}$-aryloxy ou $C_2$-$C_7$-acyloxy,

un radical hydroxy,

un radical amino, mono-($C_1$-$C_5$)-alkylamino ou di-($C_1$-$C_5$)-alkylamino,

un radical hydroxy-($C_1$-$C_5$)-alkyle, amino-($C_1$-$C_5$)alcoxy, mono- ou di-($C_1$-$C_5$)-alkylamino-($C_1$-$C_5$)-alcoxy ;

sous réserve que l'un au moins des substituants $R_1$, $R_2$, $R'_1$ et $R'_2$ soit différent de l'hydrogène ; sous forme d'isomère d ou l ou sous forme de mélange racémique.

Parmi ces composés, les composés dans lesquels les radicaux $R_1$, $R_2$, $R'_1$ et $R'_2$ représentent indépendamment un atome d'hydrogène, un radical hydroxy ou un radical méthoxy, sont particulièrement intéressants.

Il convient en outre de mentionner les composés de formule :

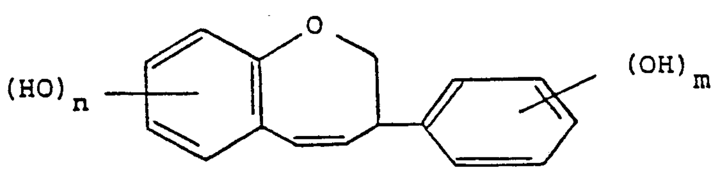

dans laquelle n est un nombre entier de 0 à 2 et m est un nombre entier de 0 à 2 sous réserve que m + n soit différent de 0.

et surtout les composés de formule :

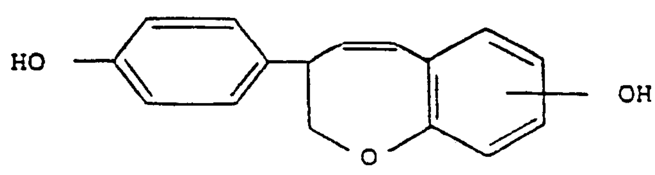

sous forme d'isomère d ou l ou sous forme de mélange racémique.

Il est important de noter que les composés selon la présente invention comportent un atome de carbone asymétrique en position 3 et peuvent donc se présenter soit sous forme d'un mélange racémique de deux formes isomères, soit sous forme lévogyre, soit sous forme dextrogyre.

Bien entendu, la présente invention concerne tant les mélanges racémiques que les isomères d ou l purs ou bien les mélanges enrichis en l'un des deux isomères.

Il est d'ailleurs évident que suivant le type de composé mis en œuvre, l'un des isomères ou bien le mélange racémique pourra présenter des propriétés plus avantageuses pour une application particulière.

Dans le cadre de la présente définition, on entend désigner par « radical alkyle » plus particulièrement des radicaux alkyle inférieur en $C_1$ à $C_5$, et de préférence des radicaux en $C_1$ à $C_3$.

Les radicaux alcoxy particulièrement préférés sont ceux qui correspondent aux radicaux alcoyles mentionnés précédemment.

Pour ce qui concerne les radicaux aryles, il s'agit plus particulièrement de radicaux carbocycliques comportant de 5 à 12 atomes dans les cycles et il s'agit de préférence du radical phényle. Les radicaux aryloxy préférés sont les radicaux correspondant aux radicaux aryles mentionnés précédemment.

Pour ce qui concerne les radicaux acyloxy, il s'agit de préférence de radicaux correspondant à des acides alcanoïques en $C_2$ à $C_7$.

La signification des autres radicaux mentionnés se déduit aisément à partir des définitions données précédemment.

La présente invention concerne également des procédés de préparation des composés selon la présente invention.

Les composés selon l'invention peuvent être préparés notamment par l'un des deux procédés suivants.

Dans un premier type de procédé, on déshydrate un composé de formule II :

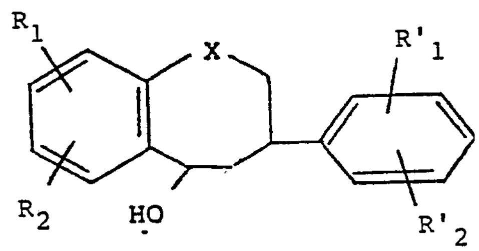

(II)

de préférence en présence d'acide p-toluène sulfonique.

Le composé de formule II peut être préparé comme cela sera explicité ci-après à partir de la cétone correspondante.

Dans un second mode de synthèse, les composés selon la présente invention sont préparés par décarboxylation d'un composé de formule III :

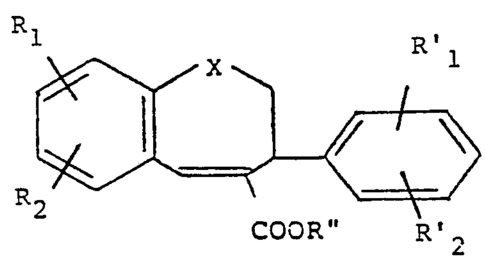

(III)

dans laquelle R" est un radical alkyle, la décarboxylation étant conduite de préférence en présence de chromite de cuivre et de quinoléine à une température comprise entre 150 et 300 °C, et de préférence à une température voisine de 200 °C.

Lorsque le composé selon la présente invention comporte des radicaux hydroxy libres, l'ensemble de la synthèse est effectué avec des radicaux hydroxy protégés sous forme de radicaux alcoxy, le radical alcoyle étant éliminé en fin de réaction afin de restituer les radicaux hydroxy libres.

Les procédés selon la présente invention conduisent en général à un composé de formule I sous forme d'un mélange racémique, qui peut être utilisé tel quel. Mais, pour certains types d'application, il peut être intéressant de séparer chacun des inverses optiques ou énantiomorphes.

Pour ce faire, on peut mettre en œuvre des procédés connus pour la séparation des énantiomorphes, en particulier par réaction du mélange racémique avec un composé optiquement actif ayant une configuration déterminée afin d'obtenir un mélange de deux diastéréoisomères qui pourront être séparés grâce à leurs propriétés physiques différentes, par exemple par cristallisation fractionnée.

Lorsque les deux diastéréoisomères sont séparés, il est possible de les redécomposer afin de restituer les deux énantiomorphes présents à l'origine dans le mélange racémique.

La nature des composés optiquement actifs appropriée dépend bien entendu du type de composé de formule I obtenu, ou des intermédiaires de synthèse précurseurs de I.

Ainsi, à titre d'exemple, lorsque le composé présente une fonction carbonyle (dérivé précurseur de l'alcool II), il sera possible d'utiliser une hydrazine optiquement active, afin de préparer une hydrazone

diastéréoisomère dédoublable notamment par cristallisation fractionnée ou technique chromatographique à haute résolution.

D'autres méthodes de résolution des mélanges racémiques peuvent être mises en œuvre, par exemple dédoublement par voie enzymatique.

Les schémas ci-après sont destinés à illustrer le mode de préparation de certains composés de départ qui peuvent être mis en œuvre dans le cadre des procédés selon la présente invention.

Schéma A

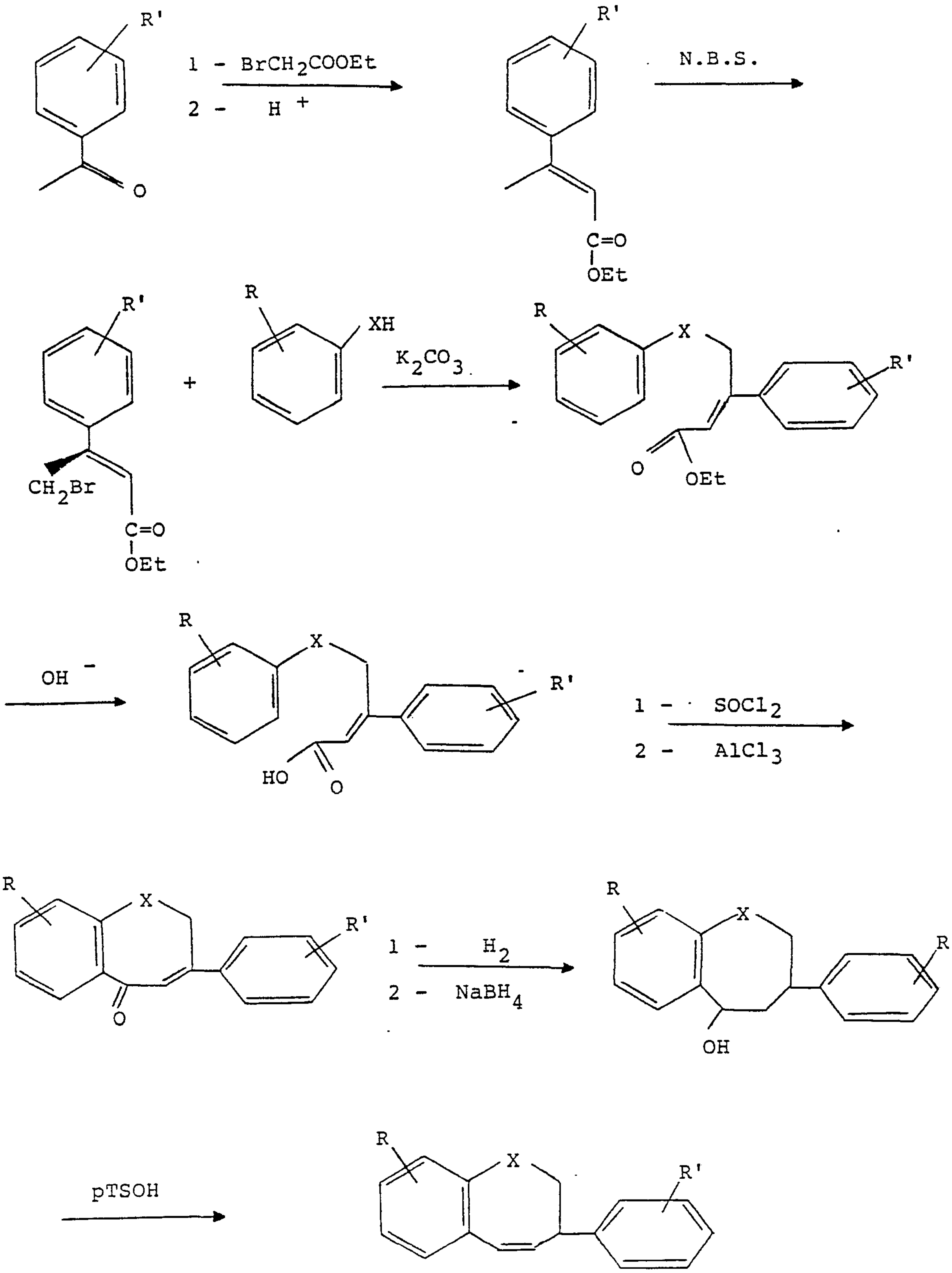

Schéma B

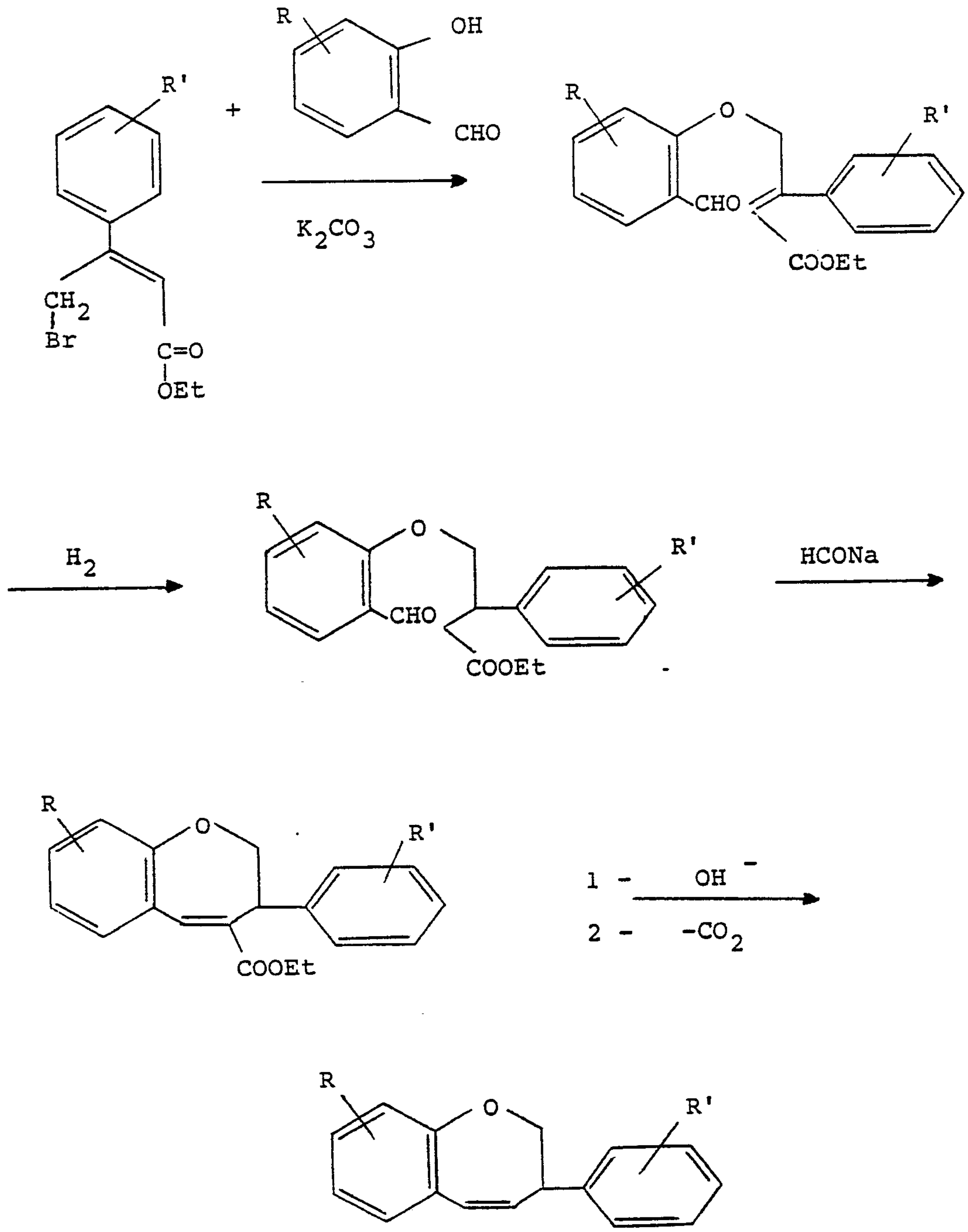


La présente invention concerne également l'utilisation des composés de formule I à titre de médicament utile dans le traitement des maladies du système cardiovasculaire et également utile pour le traitement des insuffisances veineuses, de l'inflammation et de l'oxygénation périphériques.

Sur le plan cardiovasculaire, les molécules selon la présente invention présentent une activité antihypertensive et/ou anti-angineuse. Ces nouvelles molécules ont toutes un impact modulé sur les récepteurs α-adrénergiques et présentent des propriétés oxygénatrices du sang et des tissus.

La modulation tient essentiellement à la nature de l'hétéroatome X puisque l'on démontrera ci-après que l'antagonisme sur les récepteurs postsynaptiques est plus important lorsque cet hétéroatome est l'azote que lorsqu'il s'agit, dans l'ordre, de l'oxygène et du soufre. En outre, le caractère α-lytique des molécules est plus important lorsque la position 8 est substituée par un radical hydroxy que lorsque ce même radical hydroxy se trouve en position 7.

L'activité de ces composés sur les insuffisances veineuses (syndrome post-phlébitique, hypotension orthostatique) est également intéressante de même que l'activité anti-inflammatoire et améliorant l'oxygénation périphérique (cérébrale, pulmonaire ou des membres).

Les composés selon la présente invention sont dans l'ensemble peu toxiques et leur DL O est de l'ordre de quelques grammes par kilogramme par voie orale chez la souris.

Ces composés peuvent être utilisés seuls ou en mélange avec d'autres composés pharmacologiquement actifs dans des préparations par voie entérale ou parentérale.

Les exemples ci-après sont destinés à illustrer la préparation de certains des composés selon l'invention ainsi que leur activité pharmacologique.

## Exemple 1

Préparation de la méthoxy-8 (méthoxy-4 phényl)-3 dihydro-2,3 benzoxépine-1

Ce dérivé est synthétisé selon le schéma A.

2 500 g de p.-méthoxy acétophénone traités par le bromoacétate d'éthyle en présence de zinc selon la méthode décrite par J. SCHMITT et Coll. BSCF 1966 p. 953 conduisent, après déshydratation, à l'acide p-toluène sulfonique et distillation, au (4-méthoxy phényl)-3 butène-2-oate d'éthyle (1 680 g).

Eb = 135-155 °C/0,1 mm Hg.

Cet ester est traité au reflux du tétrachlorure de carbone par la quantité stoechiométrique de N-bromosuccinimide. Après concentration et élimination du succinimide formé, on isole par cristallisation le bromo-4 (méthoxy-4 phényl)-3 butène-2 oate d'éthyle (1 550 g) qui est condensé au méthoxy-3 phénol dans l'acétone en présence de carbonate de potassium. L'ester condensé est après filtration et concentration repris dans le butanol et soumis à une hydrolyse alcaline par addition de soude. Après lavage et reprise dans l'acétate d'éthyle, on isole par cristallisation l'acide (méthoxy-3 phénoxy)-4 (méthoxy-4 phényl)-3 butène-2 oïque, 860 g.

Cet acide est traité par le chlorure de thionyle puis par le chlorure d'aluminium dans le dichlorométhane ou isolé par filtration sur colonne de silice pour obtenir un mélange de méthoxy-8 (méthoxy-4 phényl)-2 homochromène-2 one-5 et de méthoxy-8 (méthoxy-4 phényl)-3 homochromène-3 one-5 (220 g). Ce mélange, en solution dans l'acétate d'éthyle, est hydrogéné en présence de palladium/charbon sous une pression de 10 bars, réduit au borohydrure de sodium puis déshydraté en présence d'acide p-toluène sulfonique pour donner le méthoxy-8 (méthoxy-4 phényl)-3 dihydro-2,3 benzoxépine-1 (200 g) de formule :

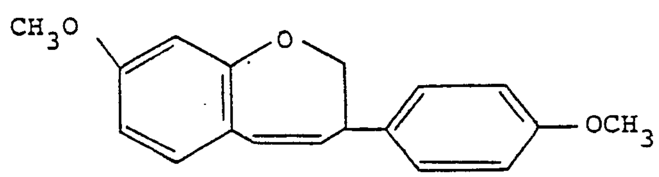

## Exemple 2

Préparation de hydroxy-8 (hydroxy-4 phényl)-3 dihydro-2,3 benzoxépine-1

Le produit obtenu à l'exemple 1, après déméthylation par chauffage à 180 °C dans le chlorhydrate de pyridinium, fournit l'hydroxy-8 (hydroxy-4 phényl)-3 dihydro-2,3 benzoxépine-1 purifié par filtration sur colonne de silice. Le produit final obtenu (110 g) possède les caractéristiques physicochimiques suivantes :

— Analyse élémentaire : C 75,25 ; H 5,57 ; O 18,95
— Poids moléculaire : 254
— Formule brute : $C_{16}$ $H_{14}$ $O_3$
— Solubilité : Ether, chloroforme, éthanol
— Spectre IR : bandes principales : 3 400, 2 900, 1 610, 1 505 $cm^{-1}$
— UV : (MeOH) max. 219 nm, 266 nm, 303 nm.
— Masse : M = 254 fragments à 147 ($C_9$ $H_7$ $O_2$) et 107 ($C_7$ $H_7$ O)
— RMN dans $CD_3OD$ : — 7 H aromatiques entre 6,3 et 7,1 ppm
— 1 H (doublet J = 12 Hz) à 6,3 ppm
— 1 H (doublet de doublet, $J_1$ = 12 Hz, $J_2$ = 5 Hz) à 5,7 ppm
— 2 H (singulet élargi) à 4,95 ppm
— 2 H (multiplet) à 4,1 ppm
— 1 H (multiplet) à 3,80 ppm ;
— RMN dérivé diacétylé dans $CDCl_3$ :
— 7 H aromatiques entre 6,6 et 7,3 ppm
— 1 H (doublet, J = 12 Hz) à 6,4 ppm
— 1 H (doublet de doublet, $J_1$ = 12 Hz, $J_2$ = 4 Hz) à 5,9 ppm
— 2H (multiplet) à 4,2 ppm

— 1 H (multiplet) à 3,9 ppm
— 6 H (singulet) à 2,2 ppm ;

— RMN $^{13}C$ (dérivé acétylé) ($CDCl_3$-TMS = 0) : 20,86 (2 q) 49,4 (d) 74,58 (t) 113,24 (d) 115,77 (d) 121,48 (2 d) 124, 19 (s) 127,90 (d) 129,25 (2 d) 132,31 (d) 133,13 (d) 138,14 (s) 150,0 (2 s) 159,56 (s) 169,03 (s) 169,27 (s).

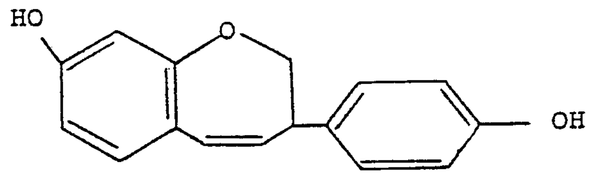

Exemple 3

Préparation du méthoxy-7 (méthoxy-4 phényl)-3 dihydro-2,3 benzoxépine-1

Ce produit est synthétisé selon le schéma B.

Le bromo-4 (méthoxy-4 phényl)-3 butène-2 oate d'éthyle, 1 500 g, tel qu'obtenu dans l'exemple 1 est condensé à l'hydroxy-2 méthoxy-5 benzaldéhyde, 788 g, dans l'acétone anhydre en présence de carbonate de potassium. Le mélange réactionnel, filtré, repris dans l'éther isopropylique est filtré sur gel de silice et le (formyl-2 méthoxy-4 phénoxy)-4 (méthoxy-4 phényl)-3 butène-2 oate d'éthyle cristallisé de l'éthanol (1 710 g).

Ce dérivé condensé est réduit dans l'acétate d'éthyle en présence de palladium/charbon sous une pression de 7 kg d'hydrogène puis purifié sur gel de silice pour donner 1 395 g de produit hydrogéné.

Celui-ci est traité par l'éthylate de sodium à reflux puis après addition d'eau, extrait au chloroforme et purifié sur gel de silice pour donner 395 g de méthoxy-7 (méthoxy-4 phényl)-3 (éthoxy-carbonyl)-4 dihydro-2,3 benzoxépine-1. Après saponification par la soude au reflux du butanol et purification sur gel de silice on isole 290 g d'acide correspondant.

Cet acide, chauffé à 210 °C en présence de chromite de cuivre et de quinoléine, se décarboxyle pour donner après purification la méthoxy-7 (méthoxy-4 phényl)-3 dihydro-2,3 benzoxépine-1 (125 g) de formule :

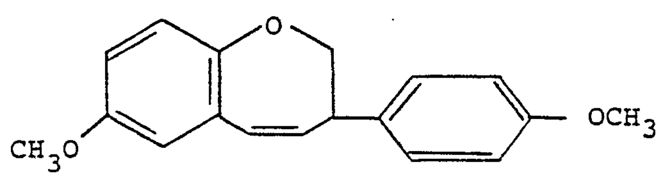

Exemple 4

Préparation du hydroxy-7 (hydroxy-4 phényl)-3 dihydro-2,3, benzoxépine-1

Le produit ci-dessus après déméthylation par chauffage à 180 °C dans le chlorhydrate de pyridinium donne l'hydroxy-7 (hydroxy-4 phényl)-3 dihydro-2,3 benzoxépine-1 (165 g).

Ce produit possède les caractéristiques physicochimiques suivantes :

— Analyse élémentaire : C 75,17 H 5,59 N 18,99
— Poids moléculaire : 254
— Formule brute : $C_{16}$ $H_{14}$ $O_3$
— Solubilité : éther, chloroforme, éthanol
— Spectre IR : bandes principales : 3 400, 2 900; 1 610, 1 505 $cm^{-1}$
— UV : (MeOH) max. 219 nm. 266 nm ; 303 nm.
— Masse : M = 254 fragments à 147 ($C_9$ $H_7$ $O_2$) et 107 ($C_7$ $H_7$ O)
— RMN dans $CD_3OD$ : — 7 H aromatiques entre 6,3 et 7,1 ppm
— 1 H (doublet, J = 12 Hz) à 6,3 ppm
— 1 H (doublet de doublet, $J_1$ = 12 Hz, $J_2$ = 5 Hz) à 5,7 ppm
— 2 H (singulet élargi) à 4,95 ppm
— 2 H (multiplet) à 4,1 ppm
— 1 H (multiplet) à 3,80 ppm ;
— HPLC : Phase $C_8$ — méthanol — eau

$$Tr \frac{\text{hydroxy-8 (hydroxy-4 phényl)-3 dihydro-2,3 benzoxépine-1}}{\text{hydroxy-7 (hydroxy-4 phényl)-3 dihydro-2,3 benzoxépine-1}} = 1$$

Exemple 5

Préparation du diéthylamino éthoxy-7 (diéthylamino éthoxy-4 phényl)-3 dihydro-2,3 benzoxépine-1

Le composé obtenu à l'exemple 4 est traité pendant 4 heures par le bromo-1 diéthylamino-2 éthane au reflux de l'acétone en présence de carbonate de potassium. On isole après chromatographie sur gel de silice le diéthylamino éthoxy-7 (diéthylamino éthoxy-4 phényl)-3 dihydro-2,3 benzoxépine-1.

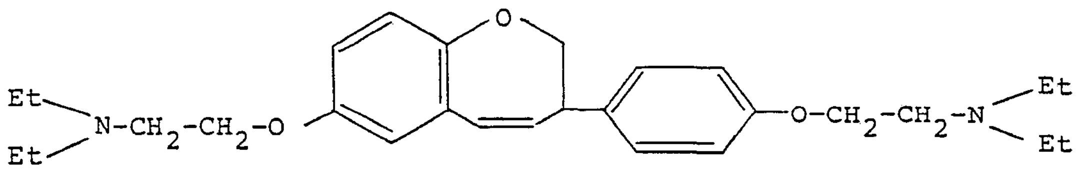

Exemple 6

Préparation de méthoxy-9 (méthoxy-4 phényl)-3 dihydro-2,3 benzoxépine-1

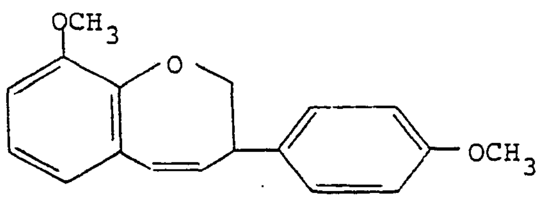

Ce composé est préparé selon le procédé de l'exemple 3 à partir de 4-méthoxy acétophénone et hydroxy-2 méthoxy-3 benzaldéhyde.

Exemple 7

Préparation de méthoxy-8 (méthoxy-3 phényl)-3 dihydro-2,3 benzoxépine-1

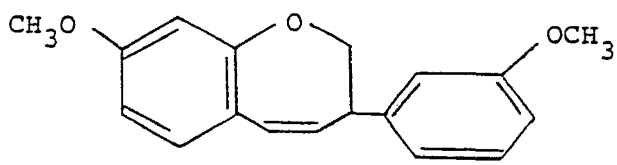

Ce composé est préparé selon le procédé de l'exemple 1 à partir de méthoxy-3 acétophénone et méthoxy-3 phénol.

Exemple 8

Préparation de diméthoxy-8,9 (méthoxy-4 phényl)-3 dihydro-2,3 benzoxépine-1

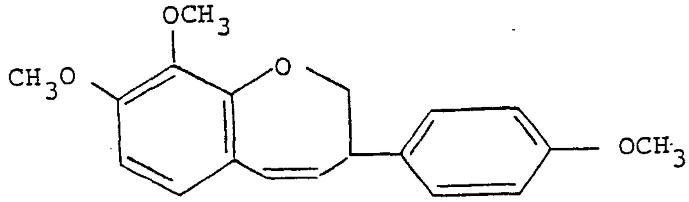

Ce composé est préparé selon l'exemple 1 à partir de méthoxy-4 acétophénone et diméthoxy-2,3 phénol.

Exemple 9

Préparation de diméthoxy-6,8 (méthoxy-4 phényl)-3 dihydro-2,3 benzoxépine-1

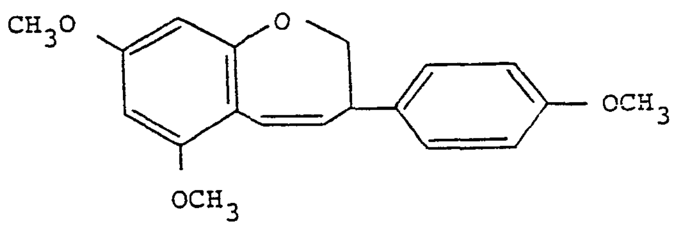

Ce composé est préparé selon le procédé de l'exemple 1 à partir de méthoxy-4 acétophénone et diméthoxy-3,5 phénol.

Exemple 10

Préparation de méthoxy-8 (diméthoxy-3,4 phényl)-3 dihydro-2,3 benzoxépine-1

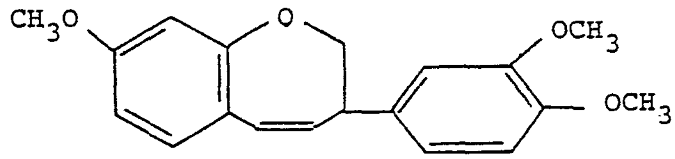

Ce composé est préparé selon le procédé de l'exemple 1 à partir de diméthoxy-3,4 acétophénone et méthoxy-3 phénol.

Exemple 11

Préparation de (méthoxy-4 phényl)-3 dihydro-2,3 benzoxépine-1

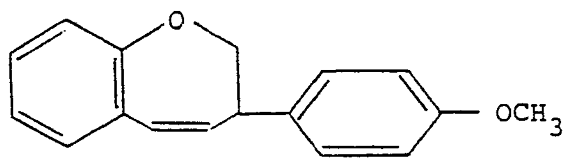

Ce composé est préparé selon le procédé de l'exemple 1 à partir de méthoxy-4 acétophénone et de phénol.

Exemple 12

Préparation de méthoxy-8 (méthoxy-4 phényl)-3 dihydro-2,3 benzothiépine-1

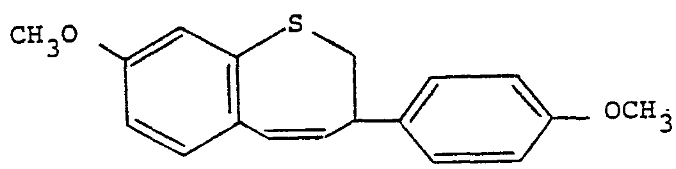

Ce composé est préparé selon le procédé de l'exemple 1 à partir de méthoxy-4 acétophénone et méthoxy-3 benzènethiol.

Exemple 13

Préparation de chloro-8 (méthoxy-4 phényl)-3 dihydro-2,3 benzoxépine-1

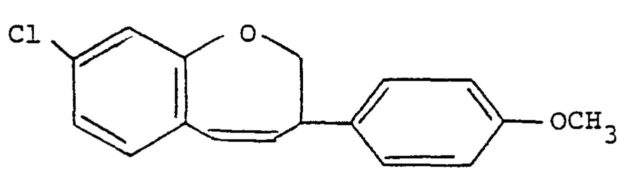

Ce composé est préparé selon le procédé de l'exemple 1 à partir de méthoxy-4 acétophénone et de chloro-3 phénol.

Exemple 14

Préparation d'amino-8 (amino-4 phényl)-3 dihydro-2,3 benzoxépine-1

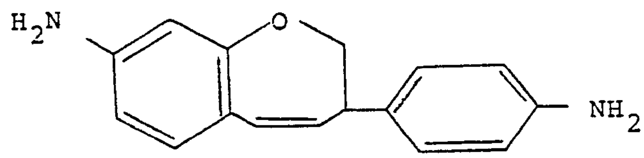

Ce composé est préparé selon le procédé de l'exemple 1 avec blocage intermédiaire de la fonction azotée, à partir d'amino-4 acétophénone et d'amino-3 phénol.

Exemple 15

Préparation de méthoxy-8 (méthoxy-4 phényl)-3 dihydro-2,3 benzazépine-1

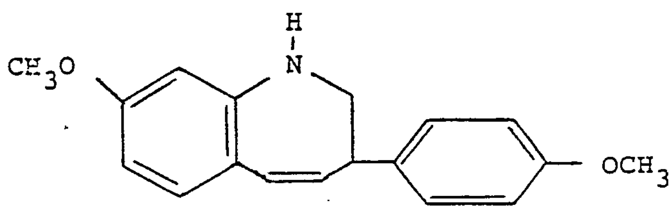

Ce composé est préparé selon le procédé de l'exemple 1 avec blocage intermédiaire de la fonction azotée, à partir de méthoxy-4 acétophénone et d'amino-3 anisole.

Les composés selon la présente invention présentent d'intéressantes propriétés anti-hypertensives et anti-angineuses.

Ces propriétés sont mises en évidence dans les exemples ci-après pour deux des composés selon l'invention :

le composé de l'exemple 2 (ci-après PM 227)

le composé de l'exemple 4 (ci-après PM 250).

Exemple 16

Activité cardiovasculaire

L'activité cardiovasculaire a été testée chez le chien par les procédés classiques mettant en œuvre la pression ventriculaire droite (PVD), la fréquence cardiaque (FC), la pression artérielle fémorale (PAF), la pression ventriculaire gauche (PVG) et sa dérive (dP/dt).

Dans le tableau 1 ci-dessous on a rassemblé les résultats moyens en pourcentage de variation par rapport à la période témoin (base 100).

Tableau 1

| | Chiens non atropinés | | | | | Chiens atropinés | | | | |
|---|---|---|---|---|---|---|---|---|---|---|
| | PVD | FC | PAF | PVG | dP/dt | PVD | FC | PAF | PVG | dP/dt |
| PM 250 0,1 mg/kg | 120 | 110 | 80 | 85 | 85 | 140 | 95 | 105 | 100 | 100 |
| PM 227 0,1 mg/kg | 110 | 95 | 104 | 105 | 103 | 120 | 90 | 90 | 105 | 102 |

Si l'on prend comme base 100 l'injection préalable de 0,5 µg/kg de noradrénaline, la modulation sur cette position apparaît nettement dans le tableau 2.

Tableau 2

| | Chiens non atropinés | | | | | Chiens atropinés | | | | |
|---|---|---|---|---|---|---|---|---|---|---|
| | PVD | FC | PAF | PVG | dP/dt | PVD | FC | PAF | PVG | dP/dt |
| PM 250 0,1 mg/kg | 95 | 115 | 110 | 100 | 100 | 100 | 95 | 85 | 95 | 95 |
| PM 227 0,1 mg/kg | 90 | 110 | 130 | 105 | 104 | 115 | 85 | 55 | 88 | 90 |

On remarque une composante muscarinique sur les récepteurs α pré-synaptiques qui apparaît pour le PM 227 et qui est très discrète pour l'autre.

Exemple 17

Propriétés oxygénatrices sanguines

Ces deux composés ont les mêmes propriétés oxygénatrices sanguines, seulement la différence apparaît dans la durée de l'activité. Le PM 227 a une activité qui se poursuit au-delà de deux heures après l'injection alors que pour le PM 250 son activité est terminée entre 1 heure et 1 heure 30.

Dans le tableau 3 ci-dessous, apparaissent la consommation d'oxygène cardiaque ($mVO_2$) et la différence artério-veineuse pulmonaire ($\Delta PO_{2p}$) pour les deux produits en base 100 par rapport à la période de référence.

Tableau 3

| Paramètres | PM 250 (0,1 mg/kg) | | | | | | | PM 227 (0,1 mg/kg) | | | | | | |
|---|---|---|---|---|---|---|---|---|---|---|---|---|---|---|
| | Temps (minutes) | | | | | | | | | | | | | |
| | 5 | 10 | 20 | 30 | 60 | 90 | 120 | 5 | 10 | 20 | 30 | 60 | 90 | 120 |
| $mVO_2$ | 90 | 85 | 85 | 90 | 95 | 100 | 100 | 80 | 75 | 75 | 90 | 85 | 78 | 80 |
| $\Delta PO_{2p}$ | 100 | 105 | 110 | 110 | 105 | 102 | 100 | 102 | 110 | 130 | 130 | 125 | 130 | 125 |

Cet exemple montre bien les modulations qu'apportent les différents substituants et leur importance relative.

De la même façon, une modulation sur l'activité anti-agrégante vis-à-vis du collagène a pu être mise en évidence mais sans que les relations structure-activité soient aussi nettes. Cette activité relativement faible (entre $10^{-5}$ et $10^{-4}$ M) n'est en fait qu'un atout supplémentaire dans les activités revendiquées plus haut au plan cardiovasculaire soit : la maladie hypertensive et les cardiopathies, plus particulièrement celle d'origine angineuse.

**Revendications**

1. Composé de formule I :

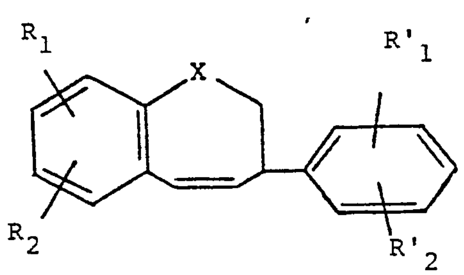

(I)

dans laquelle :

X est l'oxygène, le soufre ou >N-R', R' étant l'hydrogène ou un radical $C_1$-$C_5$ alkyle ;

$R_1$, $R_2$, $R'_1$ et $R'_2$ représentent indépendamment :

un atome d'hydrogène ou d'halogène,

un radical $C_1$-$C_5$-alkyle, $C_5$-$C_{12}$-aryle, $C_1$-$C_5$-alcoxy, $C_5$-$C_{12}$-aryloxy ou $C_2$-$C_7$-acyloxy,

un radical hydroxy,

un radical amino, mono-($C_1$-$C_5$)-alkylamino ou di-($C_1$-$C_5$)-alkylamino,

un radical hydroxy-($C_1$-$C_5$)-alkyle, amino-($C_1$-$C_5$)alcoxy, mono- ou di-($C_1$-$C_5$)-alkylamino-($C_1$-$C_5$)-alcoxy ;

sous réserve que l'un au moins des substituants $R_1$, $R_2$, $R'_1$ et $R'_2$ soit différent de l'hydrogène ; sous forme d'isomère d ou l ou sous forme de mélange racémique.

2. Composé selon la revendication 1, caractérisé en ce que les radicaux $R_1$, $R_2$, $R'_1$ et $R'_2$ représentent indépendamment un atome d'hydrogène, un radical hydroxy ou méthoxy.

3. Composé selon la revendication 2, caractérisé en ce qu'il présente la formule :

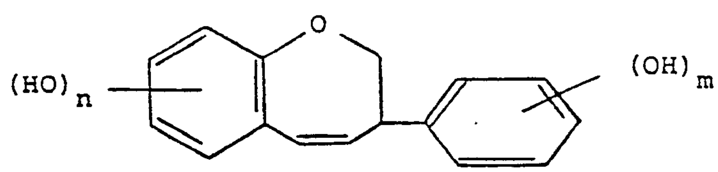

dans laquelle n est un nombre entier de 0 à 2 et m est un nombre entier de 0 à 2, sous réserve que m + n soit différent de 0.

4. Composé selon la revendication 3 de formule :

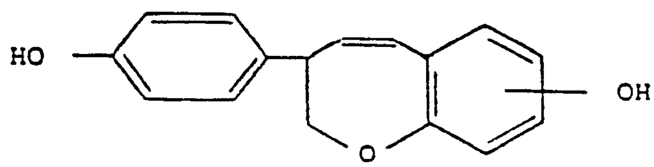

sous forme d'isomère d ou l ou sous forme de mélange racémique.

5. Procédé de préparation d'un composé selon l'une des revendications 1 à 4, caractérisé en ce qu'on déshydrate un composé de formule II :

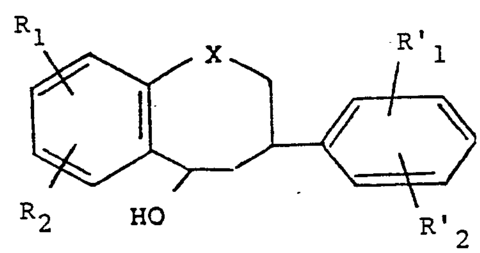

(II)

dans laquelle $R_1$, $R_2$, $R'_1$ et $R'_2$ ont les significations données dans la revendication 1.

6. Procédé selon la revendication 5, caractérisé en ce que la déshydratation est conduite en présence d'acide p-toluène sulfonique.

7. Procédé de préparation d'un composé selon l'une des revendications 1 à 4, caractérisé en ce qu'on décarboxyle un composé de formule III :

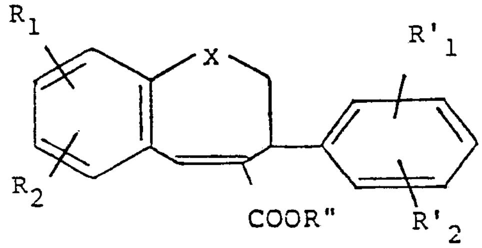

(III)

dans laquelle R" est un radical alkyle et $R_1$, $R_2$, $R'_1$ et $R'_2$ ont les significations données dans la revendication 1.

8. Procédé selon la revendication 7, caractérisé en ce qu'on décarboxyle le composé de formule III en présence de chromite de cuivre et de quinoléine à une température supérieure à 150 °C.

9. Procédé selon l'une des revendications 5 à 7, caractérisé en ce qu'on desméthyle les fonctions méthoxy pour libérer les groupements OH.

10. A titre de médicament, un composé selon l'une des revendications 1 à 4.

**Claims**

1. Compound of formula I :

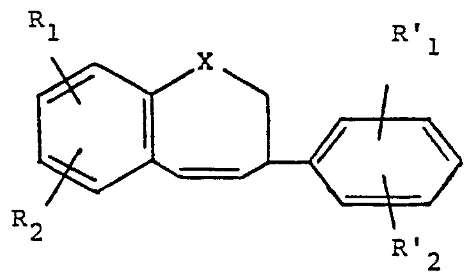

(I)

in which :

X is oxygen, sulphur or >N-R', R' being hydrogen or an alkyl radical $C_1$-$C_5$ ;

$R_1$, $R_2$, $R'_1$ and $R'_2$ represent independently :

a hydrogen or halogen atom,

an alkyl radical $C_1$-$C_5$, an aryl radical $C_5$-$C_{12}$, an alcoxy radical $C_1$-$C_5$, an aryloxy radical $C_5$-$C_{12}$ or an acyloxy radical $C_2$-$C_7$,

a hydroxy radical,

an amino radical, a mono-alkylamino radical ($C_1$-$C_5$) or di-alkylamino radical ($C_1$-$C_5$),

an alkyl-hydroxy radical ($C_1$-$C_5$), an alcoxy-amino radical ($C_1$-$C_5$), an alcoxy — ($C_1$-$C_5$) — mono — or di-alkylamino ($C_1$-$C_5$) radical ;

on condition that at least one of the substituants $R_1$, $R_2$, $R'_1$, and $R'_2$ is different from hydrogen ; in the form of a d or l isomer or in the form of a racemic mixture.

2. Compound according to claim 1, characterised in that the radicals $R_1$, $R_2$, $R'_1$ and $R_2$ represent independently a hydrogen atom, a hydroxy or methoxy radical.

3. Compound according to claim 2, characterised in that it has the formula :

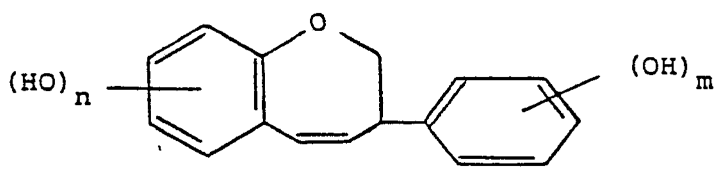

in which n is a whole number from 0 to 2 and m is a whole number from 0 to 2, on condition that m + n is different from 0.

4. Compound according to claim 3 of formula :

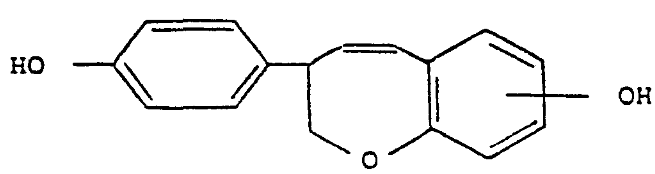

in the form of a d or l isomer or in the form of a racemic mixture.

5. Method for the preparation of a compound according to one of claims 1 to 4, characterised in that one dehydrates a compound of formula II :

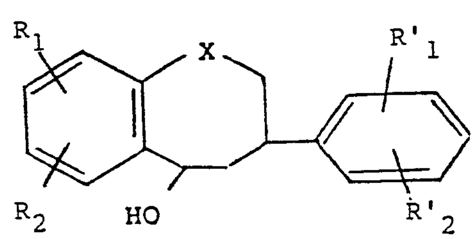

(II)

in which $R_1$, $R_2$, $R'_1$ and $R'_2$ have the meanings given in claim 1.

6. Method according to claim 5, characterised in that dehydration is carried out in the presence of p-toluene sulphonic acid.

7. Method for the preparation of a compound according to one of claims 1 to 4, characterised in that one decarboxylates a compound of formula (III) :

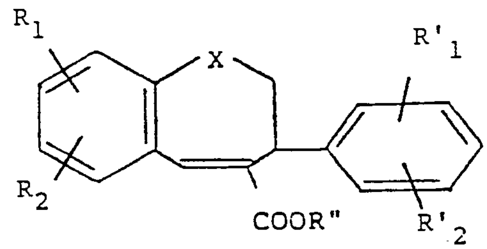

(III)

in which R" is an alkyl radical and $R_1$, $R_2$, $R'_1$ and $R'_2$ have the meanings given in claim 1.

8. Method according to claim 7, characterised in that one decarboxylates the compound of formula III in the presence of copper chromite and quinoline at a temperature higher then 150 °C.

9. Method according to one of claims 5 to 7, characterised in that one demethylates the methoxy functions in order to liberate the OH groups.

10. As a medicament, a compound according to one of claims 1 to 4.

**Patentansprüche**

1. Verbindung der Formel (I) :

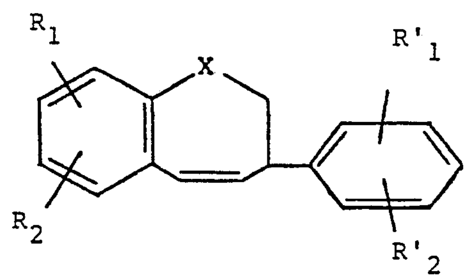

(I)

worin

X Sauerstoff, Schwefel oder N-R' ist, wobei R' Wasserstoff oder $C_1$-$C_5$-Alkyl bedeutet ;

$R_1$, $R_2$, $R'_1$ und $R'_2$ unabhängig voneinander folgende Bedeutung haben :

Wasserstoff oder Halogen ;

$C_1$-$C_5$-Alkyl, $C_5$-$C_{12}$-Aryl, $C_1$-$C_5$-Alkoxy, $C_5$-$C_{12}$-Aryloxy oder $C_2$-$C_7$-Acyloxy ;

Hydroxy ;

Amino, Mono-($C_1$-$C_5$)-alkylamino oder Di-($C_1$-$C_5$)-alkylamino ;

Hydroxy-($C_1$-$C_5$)-alkyl, Amino-($C_1$-$C_5$)alkoxy, Mono- oder Di-($C_1$-$C_5$)-alkylamino-($C_1$-$C_5$)-alkoxy ;

mit der Maßgabe, daß wenigstens einer der Substituenten $R_1$, $R_2$, $R'_1$ und $R'_2$ von Wasserstoff verschieden ist ; in Form der d- oder l-Isomeren oder in Form der racemischen Mischung.

2. Verbindung nach Anspruch 1, dadurch gekennzeichnet, daß die Reste $R_1$, $R_2$, $R'_1$ und $R'_2$ unabhängig voneinander Wasserstoff, Hydroxy oder Methoxy bedeuten.

3. Verbindung nach Anspruch 2, dadurch gekennzeichnet, daß daß sie die folgende Formel aufweist :

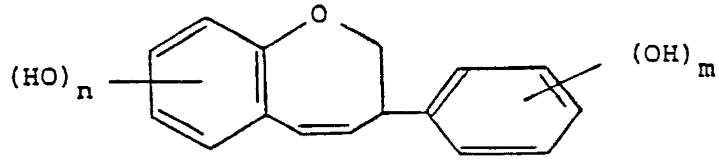

worin n eine ganze Zahl von Null bis 2 und m eine ganze Zahl von Null bis 2 ist, unter der Voraussetzung, daß m + n von Null verschieden ist.

4. Verbindung nach Anspruch 3 der Formel :

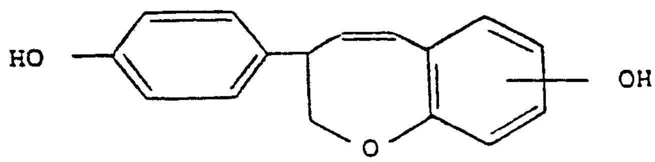

in Form des d- oder l-Isomeren oder in Form der racemischen Mischung.

5. Verfahren zur Herstellung einer Verbindung nach einem der Ansprüche 1 bis 4, dadurch gekennzeichnet, daß man eine Verbindung der Formel (II) :

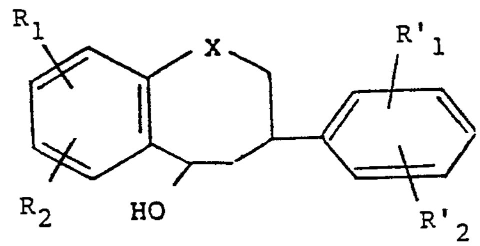

(II)

worin $R_1$, $R_2$, $R'_1$ und $R'_2$ die in Anspruch 1 angegebene Bedeutung haben, dehydriert.

6. Verfahren nach Anspruch 5, dadurch gekennzeichnet, daß die Dehydrierung in Anwesenheit von p-Toluolsulfonsäure durchgeführt wird.

7. Verfahren zur Herstellung einer Verbindung nach einem der Ansprüche 1 bis 4, dadurch gekennzeichnet, daß man eine Verbindung der Formel (III) :

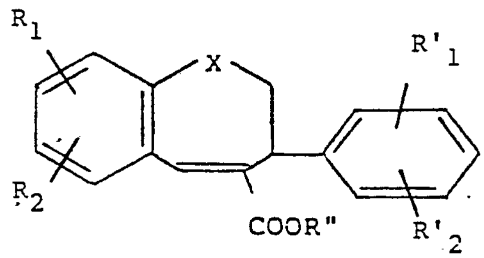

(III)

worin R" Alkyl bedeutet und $R_1$, $R_2$, $R'_1$ und $R'_2$ die in Anspruch 1 angegebene Bedeutung haben, decarboxyliert.

8. Verfahren nach Anspruch 7, dadurch gekennzeichnet, daß man eine Verbindung der Formel (III) in Anwesenheit von Kupferchromit und Chinolin bei einer Temperatur über 150 °C decarboxyliert.

9. Verfahren nach einem der Ansprüche 5 bis 7, dadurch gekennzeichnet, daß man zwecks Freisetzung der OH-Gruppe Dimethoxygruppen desmethyliert.

10. Als Medikament eine Verbindung nach einem der Ansprüche 1 bis 4.